(19) Europäisches Patentamt
European Patent Office
Office européen des brevets

(11) Veröffentlichungsnummer: **0 308 365 A1**

# (12) EUROPÄISCHE PATENTANMELDUNG

(21) Anmeldenummer: **88730228.9**

(22) Anmeldetag: **17.10.88**

(51) Int. Cl.$^4$: **C 07 J 53/00**
C 07 J 21/00, A 61 K 31/585

(30) Priorität: **17.08.83 DE 3330086**
**17.08.83 DE 3330084**
**17.08.83 DE 3330085**

(43) Veröffentlichungstag der Anmeldung:
**22.03.89 Patentblatt 89/12**

(84) Benannte Vertragsstaaten:
**AT BE CH DE FR GB IT LI LU NL SE**

(60) Veröffentlichungsnummer der früheren Anmeldung nach Art. 61 EPÜ: **0 134 529**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Nickisch, Klaus, Dr.**
**Alt-Lichtenrade 11**
**D-1000 Berlin 49 (DE)**

**Laurent, Henry, Dr.**
**Glambecker Weg 21**
**D-1000 Berlin 28 (DE)**

**Esperling, Peter**
**Furkastrasse 15C**
**D-1000 Berlin 42 (DE)**

**Bittler, Dieter**
**Bölkauer Pfad 11**
**D-1000 Berlin 27 (DE)**

**Wiechert, Rudolf, Prof. Dr.**
**Petzower Strasse 8A**
**D-1000 Berlin 39 (DE)**

**Losert, Wolfgang, Prof. Dr.**
**Landshuter Strasse 22**
**D-1000 Berlin 30 (DE)**

(54) **7Alpha-Substituierte 3-Oxo-17alpha-pregn-4-en-21.17-carbolactone, Verfahren zu ihrer Herstellung und diese enthaltende pharmazeutische Präparate.**

(57) Es werden neue 7α-substituierte 3-Oxo-17α-pregn-4-en-21.17-carbolactone der allgemeinen Formel

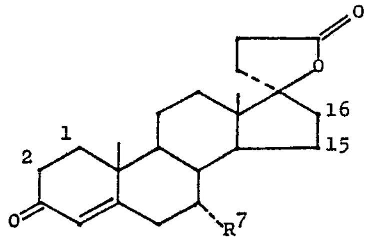

worin

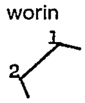

eine CC-Einfach- oder CC-Doppelbindung oder die Gruppe

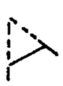

$R^7$ die Gruppe COOR″, wobei R″ eine Alkylgruppe mit 1-4 C-Atomen darstellt und

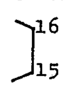

eine CC-Einfachbindung oder die Gruppe

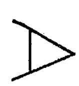

oder

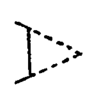

bedeuten,
ihre Herstellung und ihre Verwendung als pharmazeutische Präparate mit Antialdosteronwirkung bei verminderten endokrinologischen Nebenwirkungen beschrieben.

EP 0 308 365 A1

## Beschreibung

## 7α-SUBSTITUIERTE 3-OXO-17α-PREGN-4-EN-21.17-CARBOLACTONE, VERFAHREN ZU IHRER HERSTELLUNG UND DIESE ENTHALTENDE PHARMNAZEUTISCHE PRÄPARATE

Die Erfindung betrifft neue 7α-substituierte 3-Oxo-17α-pregn-4-en-21.17-carbolactone, Verfahren zu ihrer Herstellung und diese enthaltende pharmazeutische Präparate gemäß den Patentansprüchen.

Die neuen Verbindungen der allgemeinen Formel I haben die Eigenschaften, die Wirkung von Aldosteron oder Desoxycorticosteron auf die Natrium- und Kaliumsalzausscheidung aufzuheben bzw. umzukehren. Die erfindungsgemäßen Verbindungen sind damit geeignet zur Behandlung von bestimmten Formen der Hypertonie, von Ödemen, zum Beispiel bei Herzinsuffizienz, bei Leberzirrhose und nephrotischem Syndrom, des primären und sekundären Aldosteronismus und anderer durch Aldosteron bedingter endokrinologischer Störungen. Sie können außerdem als Diuretika eingesetzt werden.

Die neuen Wirkstoffe besitzen gegenüber dem handelsüblichen Spironolacton und seinen Metaboliten, die in 7α-Stellung statt der Acetylthio- eine Mercapto- oder Methylthiogruppe enthalten (Steroids 20 (1972) 41), den Vorteil der höheren Aktivität und der längeren Wirkungsdauer. Sie zeigen außerdem eine verminderte antiandrogene und gestagene Nebenwirkung.

Die erfindungsgemäßen Verbindungen, die wenigstens eine 1α,2α- oder 15α,16α-Methylengruppe enthalten, erweisen sich im Testmodell von Hollmann (Naunyn-Schmiedebergs Arch. Exp. Path. Pharmak. 247 (1964) 419) dem bekannten Spironolacton in ihrer Antialdosteronwirkung überraschenderweise überlegen und zeichnen sich gegenüber Spironolacton und den entsprechenden Verbindungen der deutschen Offenlegungsschrift 31 11 951, die keine 1α,2α- oder 15α,16α-Methylengruppe enthalten, durch eine geringere antiandrogene Nebenwirkung aus.

Die antiandrogene Wirkung wird bei Verbindungen festgestellt, die selber keine androgene Wirkung besitzen, aber durch ihre hohe Bindungsaffinität das körpereigene Androgen vom Rezeptor ganz oder teilweise verdrängen, wie das in gewissem Maß bei Spironolacton beobachtet wird. Es hat sich gezeigt, daß die neuen Verbindungen der allgemeinen Formel I eine geringere Affinität zum Androgenrezeptor besitzen als Spironolacton.

Im Gegensatz zum Spironolacton binden die erfindungsgemäßen Verbindungen nicht mehr an den Androgen- und Gestagenrezeptor. Das bedeutet, daß die neuen Verbindungen frei von antiandrogenen und gestagenen Nebenwirkungen sind.

Diese neuen Verbindungen der allgemeinen Formel I sind somit geeignet zur Behandlung von Ödemen, zum Beispiel bei schwerer Herzinsuffizienz, bei Leberzirrhose oder nephrotischem Syndrom sowie von Krankheitszuständen, an denen ein primärer oder sekundärer Hyperaldosteronismus beteiligt ist.

Die folgenden Verbindungen haben sich als besonders pharmakologisch wertvoll erwiesen:

1. 7α-Methoxycarbonyl-15α.16α-methylen-3-oxo-17α-pregn-4-en-21.17-carbolacton,
2. 7α-Methoxycarbonyl-1α,2α-methylen-3-oxo-17α-pregn-4-en-21,17-carbolacton.
3. 7α-Ethoxycarbonyl-1α,2α-methylen-3-oxo-17α-pregn-4-en-21,17-carbolacton.
4. 7α-Isopropyloxycarbonyl-1α,2α-methylen-3-oxo-17α-pregn-4-en-21,17-carbolacton.
5. 7α-Methoxycarbonyl-1α,2α;15ß,16ß-dimethylen-3-oxo-17α-pregn-4-en-21,17-carbolacton.
6. 7α-Ethoxycarbonyl-1α,2α;15ß,16ß-dimethylen-3-oxo-17α-pregn-4-en-21,17-carbolacton.
7. 7α-Propoxycarbonyl-1α,2α;15ß,16ß-dimethylen-3-oxo-17α-pregn-4-en-21,17-carbolacton.
8. 7α-Isopropyloxycarbonyl-1α,2α;15ß,16ß-dimethylen-3-oxo-17α-pregn-4-en-21,17-carbolacton.
9. 7α-Methoxycarbonyl-1α,2α;15α,16α-dimethylen-3-oxo-17α-pregn-4-en-21,17-carbolacton.
10. 7α-Methoxycarbonyl-15α,16α-methylen-3-oxo-17α-pregna-1,4-dien-21,17-carbolacton.

Die nachfolgende Tabelle zeigt die Antialdosteron-Wirkung an der Ratte sowie die Größe der Kompetitionsfaktoren (KF) als Maß für die Bindung am Androgen-bzw. am Gestagenrezeptor.

Hierbei bedeuten:

(+) Signifikant schlechtere Wirksamkeit der Prüfsubstanz

(++) Signifikant gleiche Wirksamkeit der Prüfusbstanz (bei 8 mg/Tier) im Vergleich zur Standardsubstanz

(+++) Signifikant bessere Wirksamkeit der Prüfsubstanz (bei 8 mg/Tier) im Vergleich zur Standardsubstanz.

TABELLE

| Verbindungs-Nr. bzw. Bezeichnung | Antialdosteron-wirkung an der Ratte | Bindung an den Androgen-Rezeptor [KF] | Bindung an den Gestagen-Rezeptor [KF] |
|---|---|---|---|
| Spironolacton (SPL) | 1 | 8,9 | 21 |
| 1 | 1,4 x SPL | 121 | 10 |
| 3 | ++ | 30 | 35 |
| 5 | 1,3 x SPL | 29 | 92 |
| 6 | 1,8 x SPL | 28 | 34 |
| 8 | 1,4 x SPL | 40 | 19 |
| 9 | 1,4 x SPL | 53 | 98 |

Die Anwendung der neuen Wirkstoffe kann in der gleicher Weise erfolgen wie beim Spironolacton. Die Dosierung der Wirkstoffe liegen unterhalb der von Spironolacton. Aufgrund der zum Teil länger anhaltenden Wirkung brauchen die neuen Wirkstoffe in der Regel nur einmal am Tag appliziert zu werden.
Die Wirkstoffe können nach an sich bekannten Methoden der Galenik zu pharmazeutischen Präparaten, vorzugsweise für die enterale Applikation, verarbeitet werden. Für die enterale Applikation kommen insbesondere Tabletten, Dragées oder Kapseln infrage, die pro Dosiseinheit etwa 50 bis 200 mg Wirkstoff in einem inerten Trägermaterial enthalten.

Die Herstellung der erfindungsgemäßen Verbindungen der allgemeinen Formel I erfolgt gemäß Anspruch 9 nach an sich bekannten Methoden.

Die Veresterung der 7α-Carboxylverbindungen der allgemeinen Formel IV erfolgt nach an sich bekannten Methoden. So kann man beispielsweise die 7α-Carbonsäure mit einen Diazaalkan, zum Beispiel Diazomethan oder Diazoethan,in einem geeigneten Lösungsmittel, wie Diethylether, Tetrahydrofuran oder Dioxan, oder einem Gemisch dieser Lösungsmittel bei einer Temperatur von 0 bis 30° C umsetzen, anschließend überschüssiges Diazaalkan durch Zusatz einer organischen Säure, wie Essig- oder Weinsäure, zersetzen und die Lösung im Vakuum vom Lösungsmittel befreien.

Die Veresterung kann aber auch so durchgeführt werden, daß man nach bekannten Methoden die Carbonsäure der allgemeinen Formel IV mit Chlorameisensäurealkylester in einem geeigneten Lösungsmittel, wie Tetrahydrofuran oder Dioxan, in Gegenwart eines tertiären Amins, wie zum Beispiel Triethylamin, bei einer Temperatur von 0 bis 30 °C zum gemischten Anhydrid (COOCO-Alkyl) umsetzt und dieses mit dem Alkohol R″OH (mit R″ in der in Formel I angegebenen Bedeutung) zum Sieden erhitzt.

Nach einer anderen Ausführungsform kann die Carbonsäure mit dem Alkohol R″OH in Gegenwart eines Kondensationsmittels wie zum Beispiel Dicyclohexylcarbodiimid direkt zum entsprechenden Ester umgesetzt werden. Auch die Umsetzung der Carbonsäure mit Halogenalkanen, wie zum Beispiel Bromethan oder Jodpropan, in Gegenwart von Silberoxid führt zu den gewünschten Estern der allgemeinen Formel I.

Die sich gegebenenfalls anschließende Einführung der $\Delta^1$-Doppelbindung kann in an sich bekannter Weise auf chemischem oder mikrobiologischem Wege erfolgen. Geeinete chemische Dehydrierungsmittel zu 1.2-Dehydrierung sind beispielsweise Selendioxid, 2.3-Dichlor-5.6-dicyanobenzochinon, Chloranil, Thalliumtriacetat oder Bleitetraacetat.

Geeignete Mikroorganismen für die 1,2-Dehydrierung sind beispeilsweise Schizomyceten, insbesondere solche der Genera Arthrobacter, wie zum Beispiel A. simplex (ATCC 6946); Bacillus, wie zum Beispiel B. lentus (ATCC 13805) oder
B. sphaericus (ATCC 7055); Pseudomonas, wie zum Beispiel P. aeruginosa (IFO 3505); Flavobacterium, wie zum Beispiel F. flavescens (IFO 3058) usw.

Die 1,2-Dehydrierung wird bevorzugt chemisch ausgeführt. Hierzu wird das 1,2-Dihydrosteroid in einem geeigneten Lösungsmittel mit dem Dehydrierungsmittel über längere Zeit auf 60 bis 120° C erhitzt. Geeignete Lösungsmittel sind beispielsweise Dioxan, Toluol, Benzol, Tetrahydrofuran, tert.Butanol und Gemische dieser Lösungsmittel. Die Reaktion ist nach mehreren Stunden beendet. Es empfiehlt sich, die Umsetzung durch Dünnschichtchromatographie zu verfolgen. Das Reaktionsgemisch wird aufgearbeitet, wenn das Ausgangsmaterial umgesetzt ist.

Die zur Durchführung des erfindungsgemäßen Verfahrens benötigten Ausgangsverbindungen sind entweder bekannt (zum Beispiel aus US-Patentschrift 4,129,564) oder können nach einem der nachstehend beschriebenen Verfahren hergestellt werden.

Herstellung der Ausgangsverbindungen

A. 1α,2α-Methylen-3-oxo-17α-pregna-4,6-dien-21,17-carbolacton:

Eine Lösung von 15.7 g Trimethylsulfoxoniumiodid in 150 ml Dimethylsulfoxid wird unter Argon mit 2.84 g einer 55prozentigen Suspension von Natriumhydrid in Mineralöl versetzt und so lange gerührt, bis eine klare Lösung entsteht. Anschließend werden 10 g 3-Oxo-17α-pregna-1,4,6-trien-21,17-carbolacton in fester Form hinzugegeben. Die Aufarbeitung erfolgt nach ca. 45 Minuten durch Eingießen in Eiswasser, das schwach mit Salzsäure angesäuert wurde. Das ausgefallene Produkt wird abfiltriert, mit Wasser gewaschen, getrocknet und danach an Kieselgel chromatographiert. Nach dem Umkristallisieren aus Aceton-Hexan erhält man 8.73 g 1α,2α-Methylen-3-oxo-17α-pregna-4,6-dien-21,17-carbolacton vom Schmelzpunkt 253.7° C. $[\alpha]_D = +176°$ (in Chloroform). UV: $\varepsilon_{282} = 21100$ (in Methanol).

B. 7α-Carboxy-15α,16α-methylen-3-oxo-17α-pregn-4-en- 21,17-carbolacton:

1. Zu einer Lösung von 3.6 g 15α,16α-Methylen-3-oxo-17α-pregna-4,6-dien-21,17-carbolacton in 50 ml absolutem Tetrahydrofuran tropft man 25 ml einer 1.8 molaren Diethylaluminiumcyanidlösung in Toluol und rührt 4 Stunden bei Raumtemperatur nach. Zur Aufarbeitung versetzt man mit 50 ml Methanol, rührt eine Stunde bei 5° C und eine Stunde bei Raumtemperatur, saugt das ausgefallene Aluminiumsalz über Kieselgel ab, wäscht mit Methanol nach und engt im Vakuum ein. Der erhaltene Rückstand wird in 30 ml Methanol aufgenommen und mit 1 g Kaliumcarbonat 1.5 Stunden gerührt. Nach dem Absaugen des Kaliumcarbonats wird im Vakuum eingeengt. Das erhaltene Rohprodukt wird durch Säulenchromatographie gereinigt. Man erhält 2.21 g 7α-Cyan-15α,16α-methylen-3-oxo-17α-pregn-4-en-21,17-carbolacton. Schmelzpunkt 260.2° C. $[\alpha]_D = +45°$ (in Chloroform). UV:$\varepsilon_{234} = 16000$ (in Methanol).

2. Zu einer auf -40° C gekühlten Lösung von 1.94 g 7α-Cyan-15α,16α-methylen-3-oxo-17α-pregn-4-en-21,17-carbolacton in 100 ml absolutem Dichlormethan tropft man 21.3 ml einer 20prozentigen Lösung von Diisobutylaluminiumhydrid in Toluol und rührt 90 Minuten nach. Zur Aufarbeitung rührt man in Kalium-Natriumtartratlösung ein, rührt 30 Minuten nach, extrahiert mit Essigester und wäscht mit Wasser neutral. Nach dem Trocknen über Magnesiumsulfat engt man im Vakuum ein. Man erhält 1.59 g 3ß,5′-Dihydroxy-15α,16α-methylen-4-androsten[(17ß-1′)-spiro-2′]perhydrofuran-7α-carbaldehyd.

3. Zu einer Lösung von 1.5 g 3ß,5′-Dihydroxy-15α,16α-methylen-4-androsten[(17ß-1′)-spiro-2′]perhydrofuran-7α-carbaldehyd in 60 ml Aceton tropft man bei -10° C 4.7 ml Joneslösung und rührt 30 Minuten bei dieser Temperatur nach. Zur Aufarbeitung versetzt man mit 0.5 ml Methanol, verdünnt mit Essigester, extrahiert die organische Phase mit verdünnter Natronlauge, wäscht die wässrige Phase mit Essigester, säuert mit Schwefelsäure an, extrahiert mit Essigester und wäscht mit halbkonzen trierter Kochsalzlösung neutral. Nach dem Trocknen über Magnesiumsulfat engt man im Vakuum ein. Man erhält 696 mg 7α-Carboxy-15α, 16α-methylen-3-oxo-17α-pregn-4-en-21,17-carbolacton. UV:$\varepsilon_{242} = 14500$ (in Methanol).

C. 7α-Carboxy-1α,2α-methylen-3-oxo-17α-pregn-4-en-21,17-carbolacton

1. Eine Lösung von 15.7 g Trimethylsulfoxoniumiodid in 150 ml Dimethylsulfoxid wird unter Argon mit 2.84 g einer 55prozentigen Suspension von Natriumhydrid in Mineralöl versetzt und so lange gerührt, bis eine klare Lösung entsteht. Anschließend werden 10 g 3-Oxo-17α-pregna-1,4,6-trien-21,17-carbolacton in fester Form hinzugegeben. Die Aufarbeitung erfolgt nach 45 Minuten durch Eingießen in Eiswasser, das schwach mit Salzsäure angesäuert wird. Das ausgefällte Produkt wird abfiltriert, mit Wasser gewaschen, getrocknet und danach an Kieselgel chromatographiert. Nach dem Umkristallisieren aus Aceton-Hexan erhält man 8.73 g 1α,2α-Methylen-3-oxo-17α-pregna-4,6-dien-21,17-carbolacton vom Schmelzpunkt 253.7° C. $[\alpha]_D = +176°$ (in Chlorofrm) UV:$\varepsilon_{282} = 21100$ (in Methanol).

2. Eine Lösung von 1.0 g 1α,2α-Methylen-3-oxo-17α-pregna-4,6-dien-21,17-carbolacton in 20 ml Dimethylformamid versetzt man mit 600 mg Kaliumcyanid, 350 mg Ammoniumchlorid und 2 ml Wasser und erhitzt 2.5 Stunden auf 100° C . Das Reaktionsgemisch wird in Eiswasser eingerührt, das ausgefällte Produkt wird abfiltriert, mit Wasser gewaschen und getrocknet. Das Rohprodukt wird an Kieselgel chromatographiert. Mit 12-15% Dichlormethan-Aceton eluiert man 980 mg, die aus Dichlormethan-Diisopropylether umkristallisiert, 587 mg 7α-Cyan-1α,2α-methylen-3-oxo-17α-pregna-4,6-dien-21,17-carbolacton ergeben. Schmp. 244.3° C. $[\alpha]_D = + 210°$ (in Chloroform) UV: $\varepsilon_{231} = 14000$ (in Methanol).

3. 3.40 g 7α-Cyan-1α,2α-methylen-3-oxo-17α-pregna-4,6-dien-21,17-carbolacton löst man in 200 ml absolutem Toluol. Die Lösung wird auf -50°C gekühlt, unter Argon tropfenweise mit 35 ml einer 20prozentigen Lösung von Diisobutylaluminiumhydrid versetzt und eine Stunde bei -40°C gerührt.

Danach wird das überschüssige Reagens durch Zugabe von 5 ml Amylalkohol zersetzt und die Reaktionsmischung in eisgekühlte Kalium-Natriumtartratlösung eingerührt. Man extrahiert mit Essigester, trocknet die organische Phase mit Natriumsulfat und verdampft das Lösungsmittel im Vakuum. Man erhält 3.41 g 3ß,5′-Dihydroxy-1α,2α-methylen-4-androsten[(17ß-1′)-spiro-2′]perhydrofuran-7α-carbaldehyd als Rohprodukt.

4. Eine Lösung von 3.41 g 3ß,5′-Dihydroxy-1α,2α-methylen-4-androsten[(17ß-1′)-spiro-2′]perhydrofuran-7α-carbaldehyd in 75 ml konzentrierter Essigsäure wird mit 10.4 ml Jones-Reagens versetzt. Nach 15 Minuten versetzt man mit 1 ml Ethanol und rührt in natriumchloridgesättigtes Eiswasser ein. Man extrahiert die organische Phase mit Natriumsulfat und verdampft das Lösungsmittel im Vakuum. Als Rückstand erhält man 3.18 g 7α-Carboxy-1α,2α-methylen-3-oxo-17α-pregn-4-en-21,17-carbolacton.

D. 7α-Carboxy-1α-2α;15ß,16ß-dimethylen-3-oxo-17α-pregn-4-en-21,17-carbolacton:

1. Eine Lösung von 5.0 g 15ß,16ß-Methylen-3-oxo-17α-pregna-4.6-dien-21,17-carbolactonin 100 ml Toluol wird mit 5.0 g 2.3-Dichlor-5.6-dicyan-1,4-benzochinon versetzt und 20 Stunden auf 80°C erhitzt. Die abgekühlte Reaktionslösung wird über Kieselgel filtriert. Man wäscht mit Diethylether nach und erhält 4.54 g eines Rohproduktes, das nochmals an Kieselgel chromatographiert wird. Mit 9-12% Aceton-Dichlormethan werden 3.88 g amorphes 15ß,16ß-Methylen-3-oxo-17α-pregna-1,4,6-trien-21,17-carbolacton eluiert. $[\alpha]_D$ +10° (in Chloroform) UV:$\varepsilon_{255}$ = 8200, $\varepsilon_{301}$ = 10800 (in Methanol).

2. Eine Lösung von 6.09 g Trimethylsulfoxoniumiodid in 58 ml Dimethylsulfoxid wird unter Argon mit 1.1 g einer 55prozentigen Suspension von Natriumhydrid in Mineralöl versetzt und 2 Stunden bei Raumtemperatur gerührt. Anschließend werden 3.88 g 15ß,16ß-Methylen-3-oxo-17α-pregna-1,4,6-trien-21,17-carbolacton in fester Form hinzugegeben. Das Reaktionsgemisch wird eine weitere Stunde gerührt und sodann in Eiswasser gegossen. Das ausgefällte Produkt wird abfiltriert, mit Wasser gewaschen und getrocknet und an Kieselgel chromatographiert. Mit 50-60% Diethylether-Dichlormethan werden 3.49 g eluiert, die aus Dichlormethan-Diisopropylether umkristallisiert, 2.25 g 1α,2α;15ß,16ß-Dimethylen-3-oxo-17α-pregna-4,6-dien-21,17-carbolacton vom Schmelzpunkt 232.4°C ergeben.
$[\alpha]_D$ = +198° (in Chloroform) UV:$\varepsilon_{282}$ = 20600 (in Methanol).

3. 4.0 g 1α,2α;15ß,16ß-Dimethylen-3-oxo-17α-pregna-4,6-dien-21,17-carbolacton löst man in 80 ml Dimethylformamid und versetzt mit 8 ml Wasser, 4.0 g Kaliumcyanid und 2.0 g Ammoniumchlorid. Das Reaktionsgemisch wird 7 Stunden bei einer Temperatur von 80°C gehalten und sodann einer Wasserfällung unterworfen. Das ausgefällte Produkt wird isoliert und an Kieselgel chromatographiert. Mit 8-10% Aceton-Dichlormethan erhält man 3.96 g 7α-Cyan-1α,2α;15ß,16ß-dimethylen-3-oxo-17α-pregn-4-en-21,17-carbolacton. Eine Probe, aus Dichlormethan-Diethylether umkristallisiert, schmilzt bei 288.2°C.
$[\alpha]_D$ = +206° (in Chloroform). UV: $\varepsilon_{232}$ = 13700 (in Methanol)

4. Eine Lösung von 3.7 g 7α-Cyan-1α,2α;15ß,16ß-dimethylen-3-oxo-17α-pregn-4-en-21,17-carbolacton in 315 ml absolutem Toluol wird bei -40°C unter Argon mit 37 ml einer 20prozentigen Lösung von Diisobutylaluminiumhydrid versetzt und 1 Stunde bei dieser Temperatur gerührt. Das Reaktionsgemisch wird unter Rühren zu 200 ml einer Natrium-Kaliumtartrat-Lösung gegeben. Anschließend wird mit Essigester extrahiert, der Extrakt wird mit Natriumsulfat getrocknet und im Vakuum eingedampft. Der Rückstand besteht aus 3.67 g 3ß,5′-Dihydroxy-1α,2α;15ß,16ß-dimethylen-4-androsten[(17ß-1′)-spiro-2′]perhydrofuran-7α-carbaldehyd.

5. 3.67 g 3ß,5′-Dihydroxy-1α,2α;15ß,16ß-dimethylen-4-androsten[(17ß-1′)-spiro-2′]perhydrofuran-7α-carbaldehyd löst man in 150 ml Aceton und versetzt die Lösung mit 11.5 ml Jones-Reagens. Nach Zugabe von 1 ml Ethanol wird in Eiswasser gegossen und mit Dichlormethan extrahiert. Die organische Phase wird mit Natriumhydrogencarbonatlösung und Wasser gewaschen, getrocknet und im Vakuum eingeengt. Man erhält 3.56 g 7α-Carboxy-1α,2α;15ß,16ß-Dimethylen-3-oxo-17α-pregn-4-en-21,17-carbolacton.

E. 7α-Carboxy-1α-2α;15α,16α-dimethylen-3-oxo-17α-pregn-4-en-21,17-carbolacton:

1. 5.0 g 15α,16α-Methylen-3-oxo-17α-pregna-4,6-dien-21,17-carbolacton werden in 50 ml Dioxan mit 5.0 g 2,3-Dichlor-5,6-dicyan-1,4-benzochinon 3 Stunden bei 100°C gerührt. Die Reaktionslösung wird dann abgekühlt, das ausgefallene Hydrochinon abgesaugt und mit Dioxan nachgewaschen. Das Filtrat wird im Vakuum weitgehend eingeengt. Der Rückstand wird in Diethylether aufgenommen, mit Natriumhydrogencarbonatlösung und Wasser gewaschen, getrocknet und eingedampft. Nach Chromatographie an Kieselgel werden 3.46 g 15α,16α-Methylen-3-oxo-17α-pregna-1,4,6-trien-21,17-carbolacton erhalten.
UV:$\varepsilon_{222}$ = 11250, $\varepsilon_{254}$ = 9140, $\varepsilon_{299}$ = 11500 (in Methanol).

2. 8.8 g Trimethylsulfoxoniumiodid werden in 99 ml Dimethylsulfoxid mit 1.39 g Natriumhydrid, eine 55prozentige Ölsuspension, bis zur Lösung des Hydrids gerührt. Dann werden unter Argon 2.8 g 15α,16α-Methylen-3-oxo-17α-pregna-1,4,6-trien-21,17-carbolacton zugegeben und 2 Stunden bei Raumtemperatur nachgerührt. Die Reaktionslösung wird in Eiswasser eingerührt, mit 2N Schwefelsäure schwach angesäuert und der ausgefallene Niederschlag abfiltriert. Nach dem Auflösen in Methylenchlorid

wird mit Wasser gewaschen, getrocknet und eingedampft. Der Rückstand wird an Kieselgel chromatographiert, und es werden 2.1 g 1α,2α;15α,16α-Dimethylen-3-oxo-17α-pregna-4,6-dien-21,17-carbolacton erhalten. UV:$\varepsilon_{281}$ = 19500 (in Methanol).

3. 1.5 g 1α,2α;15α,16α-Dimethylen-3-oxo-17α-pregna-4,6-dien-21,17-carbolacton werden in 50 ml absolutem Tetrahydrofuran mit 8.2 ml einer 1.8molaren Diethylaluminiumcyanid-Lösung in Toluol 17 Stunden unter Argon bei Raumtemperatur gerührt. Die Reaktionslösung wird anschließend in eine Kalium-Natriumtartrat-Lösung gegeben. Das durch Extraktion im Diethylether erhaltene Rohprodukt wird in 38 ml Methanol mit 45 mg Kaliumcarbonat 2 Stunden bei Raumtemperatur gerührt. Die Lösung wird mit Ether verdünnt, mit Wasser gewaschen und getrocknet. Der Rückstand wird an Kieselgel chromatographiert. Nach dem Verreiben mit Diisopropylether werden 600 mg 7α-Cyan-1α,2α; 15α,16α-dimethylen-3-oxo-17α-pregn-4-en-21,17-carbolacton mit einem Schmelzpunkt > 300° C erhalten.

4. 620 mg 7α-Cyan-1α;2α;15α,16α-dimethylen-3-oxo-17α-pregn-4-en-21,17-carbolacton werden in 115 ml Toluol mit 6.2 ml einer 2oprozentigen Diisobutylaluminiumhydrid-Lösung in Toluol 30 Minuten bei -40° C unter Argon gerührt. Nach Zugabe von 100 ml Kalium-Natriumtartratlösung wird mit Essigester extrahiert. Die organische Phase wird mit Natriumsulfat getrocknet und im Vakuum eingedampft. Der Rückstand besteht aus 650 mg 3ß,5′-Dihydroxy-1α,2α;15α,16α-dimethylen-4-androsten[(17ß-1′)-spiro-2′]perhydrofuran-7α-carbaldehyd.

5. Eine Lösung von 530 g 3ß,5′-Dihydroxy-1α,2α;15α;16α-dimethylen-4-androsten[( 17ß-1′)-spiro-2′]perhydrofuran-7α-carbaldehyd in 10.6 ml Aceton wird mit 1 ml Jones-Reagens (hergestellt aus 267 g Chrom(VI)-oxid, 230 ml konzentrierter Schwefelsäure, mit Wasser aufgefüllt auf 1000 ml) 30 Minuten bei 0° C gerührt. Man versetzt mit Dichlormethan, wäscht mit Wasser, trocknet über Natriumsulfat und engt im Vakuum ein. Als Rückstand verbleiben 520 mg 7α-Carboxy-1α,2α;15α,16α-dimethylen-3-oxo-17α-pregn-4-en-21,17-carbolacton.

Beispiel 1

Zu einer Lösung von 690 mg 7α-Carboxy-15α,16α-Methylen-3-oxo-17α-pregn-4-en-21,17-carbolacton in 10 ml Tetrahydrofuran tropft man Diazomethanlösung bis die Gelbfärbung bestehen bleibt. Man zersetzt das überschüssige Diazomethan mit Essigsäure und engt im Vakuum ein. Das erhaltene Rohprodukt wird durch Säulenchromatographie gereinigt und aus Methanol umkristallisiert. Man erhält 631 mg 7α-Methoxycarbonyl-15α,16α-methylen-3-oxo-17α-pregn-4-en-21,17-carbolacton. Schmelzpunkt 187.4° C.
$[\alpha]_D$ = + 9.1° C (in Chloroform). UV: $\varepsilon_{240}$ = 15200 (in Methanol)

Beispiel 2

Eine Lösung von 1.2 g 7α-Carboxy-1α,2α-Methylen-3-oxo-17α-pregn-4-en-21,17-carbolacton in 40 ml Tetrahydrofuran wird mit 20 ml einer aus 750 mg N-Nitrosomethylharnstoff hergestellten etherischen Diazomethanlösung versetzt. Nach 30 Minuten versetzt man mit 1 ml Essigsäure und engt im Vakuum ein. Der Rückstand wird an Kieselgel chromatographiert. Mit 30 - 47% Diethylether-Dichlormethan eluiert man 810 mg, die aus Dichlormethan-Diisopropylether umkristallisiert werden. Ausbeute:
521 mg 7α-Methoxycarbonyl-1α,2α-methylen-3-oxo-17α-pregn-4-en-21,17-carbolacton. Schmelzpunkt: 268-274° C.
$[\alpha]_D$ = +156° C (in Chloroform). UV: $\varepsilon_{238}$ = 12300 (in Methanol).

Beispiel 3

Eine Lösung von 1.2 g 7α-Carboxy-1α,2α-methylen-3-oxo-17α-pregn-4-en-21,17-carbolacton in 5 ml Dichlormethan versetzt man mit 40 mg 4-Dimethylaminopyridin, 600 mg Dicyclohexylcarbodiimid sowie mit 1 ml Ethanol. Nach 15 Minuten wird vom entstandenen N,N′-Dicyclohexylharnstoff abgesaugt und das Filtrat im Vakuum eingedampft. Der Rückstand wird an Kieselgel mehrmals chromatographiert. Mit 20 - 22 Aceton-Hexan eluiert man schließlich 460 mg, die aus Dichlormethan-Diisopropylether umkristallisiert, 322 mg 7α-Ethoxycarbonyl-1α,2α-methylen-3-oxo-17α-pregn-4-en-21,17-carbolacton vom Schmelzp. 244.8° C ergeben.
$[\alpha]_D$ = + 138° (in Chloroform). UV:$\varepsilon_{240}$ = 13400 (in Methanol).

Beispiel 4

Unter den im Beispiel 3 angegebenen Bedingungen, jedoch unter Verwendung von 2-Propanol anstelle von Ethanol, erhält man aus 1.0 g 7α-Carboxy-1α,2α-methylen-3-oxo-17α-pregn-4-en-21,17-carbolacton 293 mg 7α-Isopropyloxycarbonyl-1α,2α-methylen-3-oxo-17α-pregn-4-en-21,17-carbolacton. Schmelzpunkt: 240-242° C.
$[\alpha]_D$ = + 124° C (in Chloroform). UV:$\varepsilon_{240}$ = 13700 (in Methanol).

Beispiel 5

Unter den im Beispiel 2 angegebenen Bedingungen werden 1.30 g 7α-Carboxy-1α,2α;15ß,16ß-dimethylen-3-oxo-17α-pregn-4-en-21,17-carbolacton mit Diazomethan umgesetzt. Das Rohprodukt wird an Kieselgel chromatographiert. Mit 82-91% Essigester-Hexan werden 960 mg eluiert, die aus Dichlormethan-Diisopropylether umkristallisiert, 777 mg 7α-Methoxycarbonyl-1α,2α;15ß,16ß-dimethylen-3-oxo-17α-pregn-4-en-21,17-carbolacton vom Schmelzpunkt 260.9°C ergeben. $[\alpha]_D = +150°$ (in Chloroform). UV:$\varepsilon_{238}$ = 14000 (in Methanol).

Beispiel 6

Eine Lösung von 500 mg 7α-Carboxy-1α,2α;15ß,16ß-dimethylen-3-oxo-17α-pregn-4-en-21,17-carbolacton in 10 ml Dimethylformamid wird mit 1.0 g Silberoxid und 2 ml Bromethan versetzt und 4.5 Stunden gerührt. Die Reaktionsmischung wird filtriert und im Filtrat das Produkt mit Eiswasser gefällt. Nach Extraktion mit Dichlormethan wird die organische Phase mit Wasser gewaschen, mit Natriumsulfat getrocknet und im Vakuum zur Trockne gebracht. Der Rückstand wird an Kieselgel chromatographiert. Mit 10-12% Aceton-Dichlormethan erhält man 364 mg, die aus Dichlormethan-Diisopropylether umkristallisiert werden. Ausbeute: 274 mg 7α-Ethoxycarbonyl-1α,2α;15ß,16ß-dimethylen-3-oxo-17α-pregn-4-en-21,17-carbolacton. Schmelzpunkt: 233.3°C.
$\{\alpha]_D = +146°$ (in Chloroform).UV:$\varepsilon_{238}$ = 13600 (in Methanol).

Beispiel 7

Unter den im Beispiel 6 beschriebenen Bedingungen läßt man 700 mg 7α-Carboxy-1α,2α;15ß,16ß-dimethylen-3-oxo-17α-pregn-4-en-21,17-carbolacton mit 1-Iodpropan reagieren. Nach 30 Minuten wird aufgearbeitet und an Kieselgel chromatographiert. Mit 31-32% Aceton-Hexan eluiert man 520 mg, die aus Dichlormethan-Diisopropylether umkristallisiert, 357 mg 7α-Propoxycarbonyl-1α,2α;15ß,16ß-dimethylen-3-oxo-17α-pregn-4-en-21,17-carbolacton vom Schmelzpunkt 180.9°C ergeben.

Beispiel 8

Unter den im Beispiel 6 angegebenen Bedingungen werden 700 mg 7α-Carboxy-1α,2α;15ß,16ß-dimethylen-3-oxo-17α-pregn-4-en-21,17-carbolacton mit 2.9 ml 2-Iodpropan umgesetzt. Nach 30 Minuten wird aufgearbeitet und chromatographiert. Mit 30-32% Aceton-Hexan eluiert man 510 mg, die aus Dichlormethan-Diisopropylether umkristallisiert. 428 mg 7α-Isopropyloxycarbonyl-1α,2α;15ß,16ß-dimethylen-3-oxo-17α-pregn-4-en-21,17-carbolacton ergeben. Schmelzpunkt 214.0°C. $[\alpha]_D = +129°$ (in Chloroform). UV:$\varepsilon_{239}$ = 13500 (in Methanol).

Beispiel 9

520 mg 7α-Carboxy-1α,2α;15α,16α-dimethylen-3-oxo-17α-pregn-4-en-21,17-carbolacton werden unter den im Beispiel 2 beschriebenen Bedingungen mit Diazomethan zur Reaktion gebracht. Das Rohprodukt wird chromatographiert und aus Diisopropylether umkristallisiert. Ausbeute: 272 mg 7α-Methoxycarbonyl-1α,2α;15α,16α dimethylen-3-oxo-17α-pregn-4-en-21,17-carbolacton. Schmelzpunkt: 237.3°C. UV:$\varepsilon_{238}$ = 13400 (in Methanol).

Beispiel 10

Eine Lösung von 500 mg 7α-Methoxycarbonyl-15α,16α-methylen-3-oxo-17α-pregn-4-en-21,17-carbolacton in 10 ml Dioxan wird mit 500 mg 2,3-Dichlor-5,6-dicyan-1,4-benzochinon versetzt und 16 Stunden zum Sieden erhitzt. Nach dem Abkühlen wird filtriert und im Vakuum eingeengt. Das Rohprodukt wird an Kieselgel mit Dichlormethan/Aceton chromatographiert. Man erhält 220 mg 7α-Methoxycarbonyl-15α,16α-methylen-3-oxo-17α-pregna-1,4-dien-21,17-carbolacton. UV:$\varepsilon_{241}$ = 16700 (in Methanol).

Beispiel 11

205 mg 7α-Methoxycarbonyl-15α,16α-methylen-3-oxo-17α-pregn-4-en-21,17-carbolacton werden in 2 ml Methanol suspendiert und mit 28 mg Kaliumhydroxid in 0,5 ml Wasser 16 Stunden bei Raumtemperatur und 1 Stunde bei 60° C gerührt und im Vakuum eingeengt. Das erhaltene Öl wird in wenig Ethanol gelöst und mit Diethylether gefällt. Man erhält 120 mg 17ß-Hydroxy-7α-methoxycarbonyl-15α,16α-methylen-3-oxo-17α-pregn-4-en-21-carbonsäure-Kaliumsalz.

**Patentansprüche**

1. 7α-Substituierte 3-Oxo-17α-pregn-4-en-21.17-carbolactone der allgemeinen Formel I

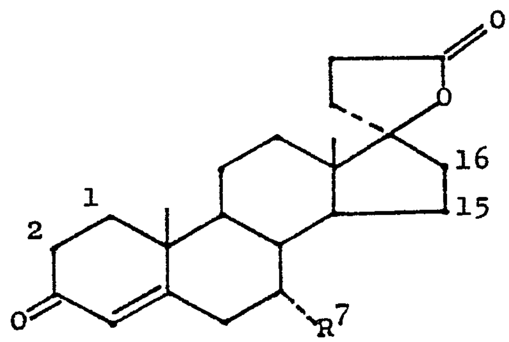

(I),

worin

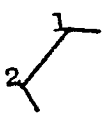

eine CC-Einfach- oder CC-Doppelbindung oder die Gruppe

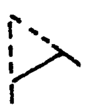

$R^7$ die Gruppe COOR″, wobei R″ eine Alkylgruppe mit 1-4 C-Atomen darstellt und

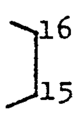

eine CC-Einfachbindung oder die Gruppe

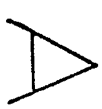 oder 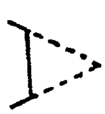

bedeuten.

2. 7α-Methoxycarbonyl-15α.16α-methylen-3-oxo-17α-pregn-4-en-21.17-carbolacton
3. 7α-Ethoxycarbonyl-1α.2α-methylen-3-oxo-17α-pregn-4-en-21.17-carbolacton
4. 7α-Methoxycarbonyl-1α.2α;15β.16β-dimethylen-3-oxo-17α-pregn-4-en-21.17-carbolacton
5. 7α-Ethoxycarbonyl-1α.2α;15β.16β-dimethylen-3-oxo-17α-pregn-4-en-21.17-carbolacton
6. 7α-Isopropyloxycarbonyl-1α.2α;15β.16β-dimethylen-3-oxo-17α-pregn-4-en-21.17-carbolacton
7. 7α-Methoxycarbonyl-1α.2α;15α.16α-dimethylen-3-oxo-17α-pregn-4-en-21.17-carbolacton

8. Pharmazeutische Präparate, gekennzeichnet durch einen Gehalt an Verbindungen gemäß den Ansprüchen 1 bis 7.

9. Verfahren zur Herstellung von 7α-substituierten 3-Oxo-17α-pregn-4-en-21.17-carbolactonen der allgemeinen Formel I

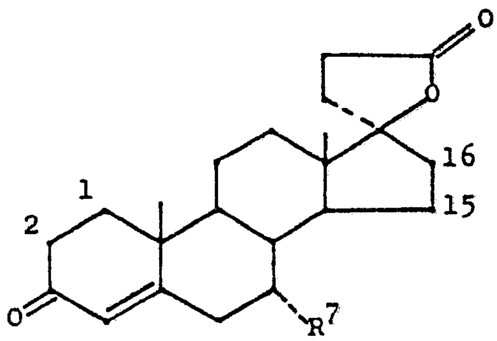

(I),

worin

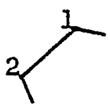

eine CC-Einfach- oder CC-Doppelbindung oder die Gruppe

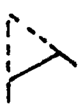

$R^7$ die Gruppe COOR″, wobei R″ eine Alkylgruppe mit 1-4 C-Atomen darstellt und

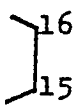

eine CC-Einfachbindung oder die Gruppe

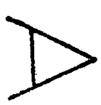

oder

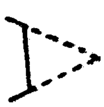

bedeuten,
dadurch gekennzeichnet, daß man eine 7α-Carboxyl-Verbindung der allgemeinen Formel IV

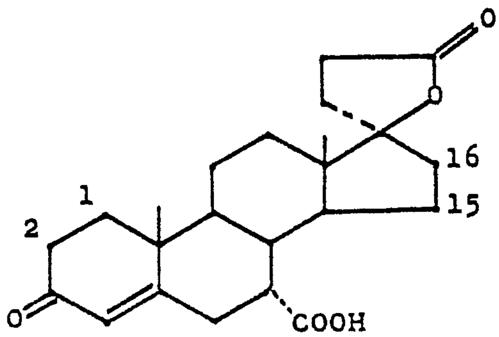 (IV),

worin

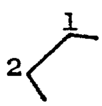 und 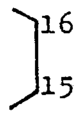

die in Formel I angegebene Bedeutung haben, zu einem $C_1$-$C_4$-Alkylester umsetzt.

Europäisches Patentamt

# EUROPÄISCHER RECHERCHENBERICHT

Nummer der Anmeldung

EP 88 73 0228

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 061 418 (SCHERING AG)<br>* Ansprüche *<br>--- | 1,8,9 | C 07 J 53/00<br>C 07 J 21/00<br>A 61 K 31/585 |
| A | US-A-3 787 396 (R. M. WEIER)<br>* Ansprüche; Spalten 1,2 *<br>----- | 1,8,9 | |
| | | | **RECHERCHIERTE SACHGEBIETE (Int. Cl.4)** |
| | | | C 07 J 53/00 |

Der vorliegende Recherchenbericht wurde für alle Patentansprüche erstellt

| Recherchenort | Abschlußdatum der Recherche | Prüfer |
|---|---|---|
| DEN HAAG | 12-12-1988 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)